# EUROPEAN PATENT SPECIFICATION

(11) **EP 3 035 990 B1**
(45) Date of publication and mention of the grant of the patent: **22.05.2019**
(21) Application number: 14761491.1
(22) Date of filing: 21.08.2014
(51) Int. Cl.: A61M 25/00

(54) **CATHETERS AND CATHETER SHAFTS**
KATHETER UND KATHETERSCHÄFTE
CATHÉTERS ET TIGES DE CATHÉTER

(30) Priority: 23.08.2013 US 201361869277 P
(43) Date of publication of application: 29.06.2016
(73) Proprietor: Boston Scientific Scimed, Inc., Maple Grove, MN 55311 (US)
(72) Inventor: STORBECK, Gene T., Franklin MA 02038-1726 (US); PATEL, Jay R., Acton, Massachusetts 01720 (US); NADONE, Christopher L., South Grafton, Massachusetts 01560 (US); PATEL, Mayur Kiran, Framingham, Massachusetts 01701 (US)
(74) Representative: Vossius & Partner Patentanwälte Rechtsanwälte mbB
(86) International application number: PCT/US2014/052149
(87) International publication number: WO 2015/027094

(56) References cited:
- EP-A2- 0 807 446
- WO-A1-2012/116337
- US-A- 5 702 373

## Description

### Technical Field

The present disclosure pertains to medical devices, and methods for manufacturing medical devices. More particularly, the present disclosure pertains to elongated intracorporeal medical devices including a tubular member connected with other structures, and methods for manufacturing such devices.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

Patent document WO 2012/116337 A1 discloses: a medical device, comprising: a catheter shaft having an inner surface defining a lumen; a liner disposed within the lumen and along the inner surface; wherein at least a section of the liner is radially spaced from the inner surface of the catheter shaft so that a space is defined therebetween; wherein the liner includes an inner layer, an outer layer, and a reinforcing member.

Furthermore patent document US 5,702,373 discloses: a medical device, comprising: a catheter shaft having an inner surface defining a lumen; a liner disposed within the lumen and along the inner surface; wherein the liner includes an inner layer, an intermediate layer, and an outer reinforcing member.

### Brief Summary

The aforementioned objectives are attained by means of a respective medical device according to claims 1 and 11.

This disclosure provides design, material, manufacturing method, and use alternatives for medical devices. An example medical device may include a catheter shaft having an inner surface defining a lumen. A liner may be disposed within the lumen and along the inner surface. At least a portion of the liner may be radially spaced from the inner surface of the catheter shaft so that a space is defined therebetween. The liner may include an inner layer, an outer layer, and a polymeric reinforcing member. At least a portion of the polymeric reinforcing member may be disposed between an outer surface of the inner layer and an outer surface of the outer layer. The polymeric reinforcing member may have a melting temperature lower than the melting temperature of the inner layer, the outer layer, or both.

An example catheter shaft may include an inner polymeric layer, an outer polymeric layer, and a polymeric reinforcement at least partially embedded within the outer polymeric layer. The polymeric reinforcement may have a melting temperature lower than the melting temperature of the inner polymeric layer, the outer polymeric layer, or both.

Another example medical device may include a catheter shaft having an inner surface defining a lumen. A liner may be disposed within the lumen and along the inner surface. At least a portion of the liner may be radially spaced from the inner surface of the catheter shaft so that a space is defined therebetween. The liner may include an inner layer, an intermediate layer, and an outer polymeric reinforcing member. The polymeric reinforcing member may have a melting temperature lower than the melting temperature of the inner layer, the intermediate layer, or both.

Another example catheter shaft may include an inner polymeric layer, an intermediate polymeric layer, and an outer polymeric reinforcement at least partially embedded within the intermediate polymeric layer. The outer polymeric reinforcement may have a melting temperature lower than the melting temperature of the inner polymeric layer, the intermediate polymeric layer, or both.

An example method for manufacturing a medical device may include extruding a catheter shaft. The catheter shaft may include an inner layer, an intermediate layer, and an outer polymeric reinforcing layer. Extruding the catheter shaft may include heating the catheter shaft. Heating the catheter shaft may cause the outer polymeric reinforcing layer to become at least partially embedded within the intermediate layer.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
Figure 1 is a side view of an example medical device;
Figure 2 is a cross-sectional view taken through line 2-2 in Figure 1;
Figure 3 is a cross-sectional view of an inventive medical device;
Figure 4 is a cross-sectional view of another example medical device; and
Figure 5 is a cross-sectional view of another inventive medical device.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the claims.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

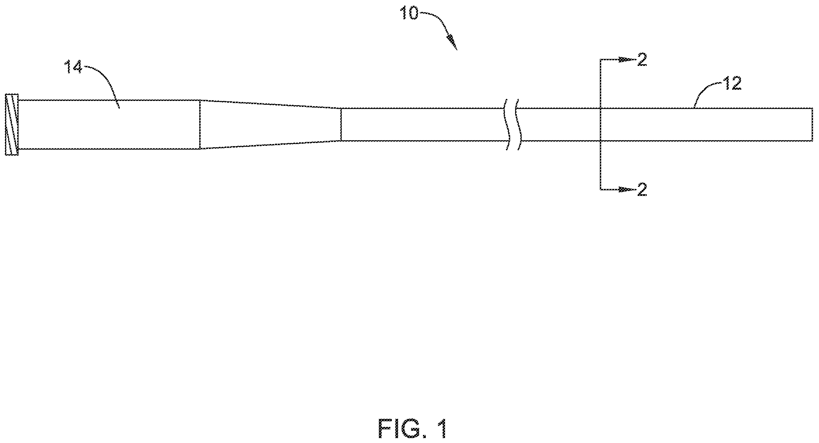

Figure 1 is a side view of an example medical device 10. Medical device 10 may include a catheter shaft 12. A hub 14 may be attached to catheter shaft 12. Medical device 10 may be used for a number of different interventions. For example, medical device 10 may be a catheter that can be used for intravascular procedures (including cardiac procedures, peripheral procedures, and neural procedures). In some examples, medical device 10 may also be used to inject contrast materials (e.g., including injection of contrast materials at relatively high pressures), deliver embolic agents (e.g., coils, microspheres, particles with low friction, etc.), and the like.

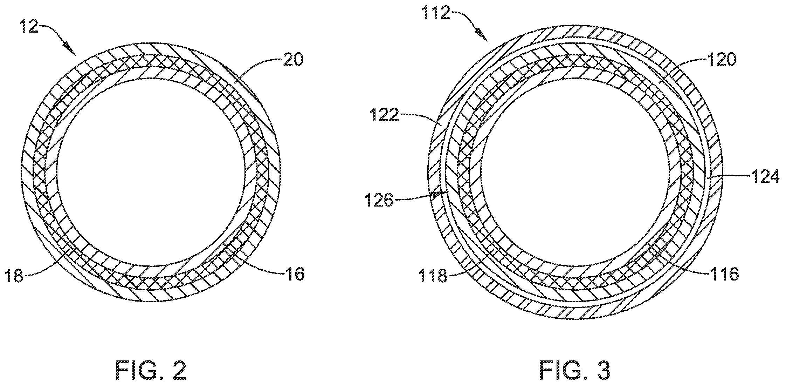

In at least some examples, catheter shaft 12 may include a plurality of layers. For example, Figure 2 illustrates that catheter shaft 12 may include an inner liner or layer 16, a reinforcing layer 18, and an outer layer 20. Liner 16 may include lubricious material such as polytetrafluoroethylene (PTFE), etched PTFE, fluorinated ethylene propylene (FEP), or the like. Other materials are contemplated including those disclosed herein. Outer layer 20 may include one or more polymers such as polyether block amide, polyurethane, combinations or blends thereof, or the like. Other materials are contemplated including those disclosed herein. All of the layers 16/18/20 may extend along the full length of catheter shaft 12. Alternatively, one or more of layers 16/18/20 may extend along only a portion of the length of catheter shaft 12.

Reinforcing layer 18 may include a braid, coil, mesh, or other suitable reinforcement. In at least some examples, reinforcing layer 18 may include a polymeric braid. For example, reinforcing layer 18 may include an ultra-high molecular weight polyethylene braid. Other materials and/or reinforcements are contemplated including those disclosed herein. In at least some examples, the melting temperature of reinforcing layer 18 may be less than the melting temperature of liner 16, outer layer 20, or both.

Manufacturing catheter shaft 12 may include a number of different methods. For example, liner 16 may be disposed on a mandrel. The mandrel may vary in size, depending on the intervention. For example, the mandrel may be a silver coated copper core or other suitable mandrel with an outer diameter in the range of about 0.25 to 1.3 mm (0.01 to 0.05 inches), or about 0.5 to 1.0 mm (0.02 to 0.04 inches), or about 0.56 to 0.69 mm (0.022 to 0.027 inches) or so. In some examples, reinforcing layer 18 may be disposed along the outer surface of liner 16 and outer layer 20 may be disposed along the outer surface of reinforcing layer 18. In other examples, outer layer 20 may be disposed along the outer surface of liner 16 and reinforcing layer 18 may be disposed along the outer surface of outer layer 20. The process for disposing layers 16/18/20 onto the mandrel may include an extrusion process. When using an extrusion process, the assembly may be subjected to extrusion temperatures in the range of about 100 to 200°C, or about 120 to 190°C, or about 140 to 170°C. Under such conditions, reinforcing layer 18 may become embedded and/or at least partially embedded within outer layer 20. For example, at least a portion of outer layer 20 may be disposed radially outward of the outer surface of reinforcing layer 18. In some instances, reinforcing layer 18 may become disposed at or near the inner surface of outer layer 20 so that reinforcing layer 18 is essentially positioned between liner 16 and outer layer 20. In some of these and in other examples, portions of outer layer 20 may be interlocked with or otherwise disposed within the interstices of reinforcing layer 18. This may form or define a "composite layer" that includes both the material of reinforcing layer 18 and the material of outer layer 20.

The process for manufacturing catheter shaft 12 (e.g., the extrusion process) may allow for relatively thin catheter shafts 12 to be manufactured. For example, catheter shaft 12 may have a wall thickness as low as about 0.0025 to 0.025 mm (0.0001 to 0.001 inches), or about 0.0025 to 0.005 mm (0.0001 to 0.0002 inches), or about 0.0038 mm (0.00015 inches). In general, the process may allow for catheter shafts 12 to be manufactured having relatively larger inner diameters while still maintaining relatively small outer diameters. The process may also result in relatively strong catheter shafts 12. This may be due at least in part to the use of ultra-high molecular weight polyethylene (e.g., in reinforcing layer 18), which may have relatively tenacious fibers. For example, catheter shaft 12 may have a tensile strength capable of withstanding forces up to about 13.6 - 27.1 Nm (10-20 foot-pounds), or up to about 16.3 - 24.4 Nm, (12-18 foot-pounds), or up to about 21.7 Nm (16 foot-pounds). Catheter shaft 12 may be capable of withstanding pressures exceeding 5.5 MPa (800 psi), or exceeding 6.9 MPa (1000 psi), or exceeding 8.3 Mpa (1200 psi). These are just examples. The presence of reinforcing layer 18 may also provide catheter shaft 12 with enhanced cut resistance, tear resistance, kink resistance, etc. These features may be further enhanced when reinforcing layer 18 is positioned at or near the outer surface of catheter shaft 12 (e.g., including examples where reinforcing layer 18 is the outer layer of the catheter shaft as disclosed herein).

Other structural features and/or variations are contemplated for medical device 10 and/or catheter shaft 12. For example, in some instances medical device 10 may be used for the embolization of tumors and/or hemorrhagic conditions. According to these examples, a glue (e.g., a cyanoacrylate or other suitable glue) may be administered through catheter shaft 12 to a target site. When using a cyanoacrylate or other glue, the glue could polymerize earlier than desired, which may essentially "glue" catheter shaft 12 to the anatomy. To reduce unwanted premature polymerization, the cyanoacrylate may be mixed with an ethiodized oil (e.g., ethiodol, thiodol, lipiodol, or the like). In some of these and in other examples, to reduce unwanted premature polymerization, the distal portion or tip of catheter shaft 12 may be coated with a material that may influence (e.g., reduce) polymerization. For example, the distal portion or tip of catheter shaft 12 may include an outer PTFE coating. Other coatings may also be used including polyethylene coatings, polypropylene coatings, or the like.

In still further examples, the distal portion or tip of catheter shaft 12 may be designed to be separable from catheter shaft 12 in the event that catheter shaft 12 is unintentionally glued to the anatomy. For example, the distal tip may include a perforation, a line of weakness, a scoring, a thinning, or other structural feature that allows the distal tip of catheter shaft 12 to separate from catheter shaft 12 in the event that catheter shaft 12 is unintentionally glued to the anatomy. Separating the distal tip from catheter shaft 12 may include application of force to catheter shaft 12 so that distal tip "breaks off' or otherwise separates from catheter shaft 12. In at least some examples, the detachable distal tip of catheter shaft 12 may include a biocompatible or bioresporable material so that the distal tip may be essentially permanently implanted, if desired.

An inventive catheter shaft 112 is illustrated in Figure 3 that may be similar in form and function to other catheter shafts disclosed herein. Here it can be seen that catheter shaft 112 includes an inner liner 126. Liner 126 includes inner layer 116, reinforcing layer 118, and outer layer 120. According to the invention, liner 126 has a form resembling catheter shaft 12. Catheter shaft 112 includes a tubular member 122. In at least some embodiments, tubular member 122 may be a metallic or polymeric tube and may include any of the materials disclosed herein. For example, tubular member 122 may include a nickel-titanium alloy tube. In some embodiments, tubular member 122 may have a plurality of slots (not shown) formed therein.

As shown, liner 126 is disposed within tubular member 122. According to the invention, tubular member 122 is radially spaced from liner 126 so that a gap or space 124 is defined therebetween. Space 124 may extend along only a portion of the length of catheter shaft 112 or along essentially the entire length. In some embodiments, liner 126 is free from direct attachment to tubular member 122. For example, the proximal end of liner 126 and the proximal end of tubular member 122 may both be attached to hub (e.g., hub 14). At the distal end of catheter shaft 112, a region of liner 126 may extend distally beyond the distal end of tubular member 122. In some embodiments, a sleeve (not shown) may be disposed along the outer surface of tubular member 122 and the outer surface of liner 126 adjacent to the distal end of tubular member 122 and/or the junction at the distal end of tubular member 122 and liner 126. The sleeve may bond to both tubular member 122 and liner 126 so as to secure together these structures.

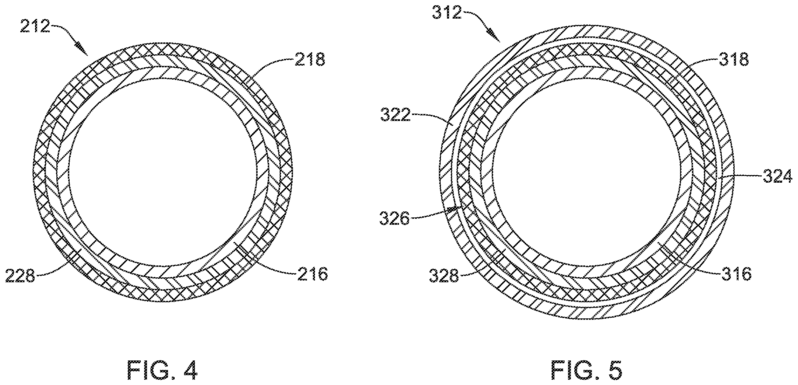

Figure 4 illustrates another example catheter shaft 212 that may be similar in form and function to other catheter shafts disclosed herein. Catheter shaft 212 may include inner liner 216, intermediate layer 228, and reinforcing layer 218. Liner 216 may be similar to other liners disclosed herein including liner 16. Similarly, intermediate layer 228 may similar to other layers disclosed herein including outer layer 20. Likewise, reinforcing layer 218 may be similar to other reinforcing layers disclosed herein including reinforcing layer 18.

Reinforcing layer 218 may be positioned along the outer surface of catheter shaft 212. This may be desirable for a number of reasons. For example, having reinforcing layer 218 along the outer surface of catheter shaft 212 may provide catheter shaft 212 with enhanced cut resistance, tear resistance, kink resistance, etc.

Another inventive catheter shaft 312 is illustrated in Figure 5 that may be similar in form and function to other catheter shafts disclosed herein. Here it can be seen that catheter shaft 312 includes inner liner 326. Liner 326 includes inner layer 316, intermediate layer 328, and reinforcing layer 318. Catheter shaft 312 includes a tubular member 322. Tubular member 322 is radially spaced from liner 326 so that a gap or space 324 is defined therebetween. Space 324 may extend along only a portion of the length of catheter shaft 312 or along essentially the entire length. In some embodiments, liner 326 is free from direct attachment to tubular member 322. For example, the proximal end of liner 326 and the proximal end of tubular member 322 may both be attached to hub (e.g., hub 14). At the distal end of catheter shaft 312, a region of liner 326 may extend distally beyond the distal end of tubular member 322. In some embodiments, a sleeve (not shown) may be disposed along the outer surface of tubular member 322 and the outer surface of liner 326 adjacent to the distal end of tubular member 322 and/or the junction at the distal end of tubular member 322 and liner 326. The sleeve may bond to both tubular member 322 and liner 326 so as to secure together these structures.

The materials that can be used for the various components of medical device 10 (and/or other medical devices and/or catheter shafts disclosed herein) may include those commonly associated with medical devices. For simplicity purposes, the following discussion makes reference to catheter shaft 12 and other components of medical device 10. However, this is not intended to limit the devices and methods described herein, as the discussion may be applied to other similar tubular members and/or components of tubular members or devices disclosed herein.

Catheter shaft 12 and/or other components of medical device 10 may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b*-styrene) (for example, SIBS and/or SIBS 50A), polycarbonates, ionomers, biocompatible polymers, other suitable materials, or mixtures, combinations, copolymers thereof, polymer/metal composites, and the like. In some examples the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some examples, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some examples, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some examples, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some examples, the linear elastic and/or non-super-elastic nickel-titanium alloy may be in the range of about 50 to about 60 weight percent nickel, with the remainder being essentially titanium. In some examples, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM™ (available from Neo-Metrics) and GUM METAL™ (available from Toyota). In some other examples, a superelastic alloy, for example a superelastic nitinol can be used to achieve desired properties.

In at least some examples, portions or all of catheter shaft 12 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of medical device 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of medical device 10 to achieve the same result.

In some examples, a degree of Magnetic Resonance Imaging (MRI) compatibility is imparted into medical device 10. For example, catheter shaft 12, or portions thereof, may be made of a material that does not substantially distort the image and create substantial artifacts (i.e., gaps in the image). Certain ferromagnetic materials, for example, may not be suitable because they may create artifacts in an MRI image. Catheter shaft 12, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A medical device (10), comprising:
a catheter shaft (112) having an inner surface defining a lumen;
a liner (126) disposed within the lumen and along the inner surface;
wherein at least a section of the liner (126) is radially spaced from the inner surface of the catheter shaft (112) so that a space is defined therebetween;
wherein the liner (126) includes an inner layer (116), an outer layer, and a polymeric reinforcing member (118);
wherein at least a portion of the polymeric reinforcing member (118) is disposed between an outer surface of the inner layer (116) and an outer surface of the outer layer;
wherein the polymeric reinforcing member (118) has a melting temperature lower than the melting temperature of the inner layer (116) and/or the outer layer; and
wherein the polymeric reinforcing member (118) is at least partially embedded within the outer layer of the liner (126).

2. The medical device (10) of claim 1, wherein the catheter shaft (112) includes a nickel-titanium alloy.

3. The medical device (10) of any one of claims 1-2, wherein the catheter shaft (112) has a plurality of slots formed therein.

4. The medical device (10) of any one of claims 1-3, wherein the liner (126) is free from attachment with the inner surface of the catheter shaft (112).

5. The medical device (10) of any one of claims 1-4, wherein the inner layer (116) of the liner (126) includes polytetrafluoroethylene.

6. The medical device (10) of any one of claims 1-5, wherein the inner layer (116) of the liner (126) includes fluorinated ethylene propylene.

7. The medical device (10) of any one of claims 1-6, wherein the outer layer of the liner (126) includes polyether block amide.

8. The medical device (10) of any one of claims 1-7, wherein the outer layer of the liner (126) includes a blend of polyether block amide and polyurethane.

9. The medical device (10) of any one of claims 1-8, wherein the polymeric reinforcing member (118) includes a polymeric braid.

10. The medical device (10) of any one of claims 1-9, wherein the polymeric reinforcing member (118) includes ultra-high molecular weight polyethylene braid.

11. A medical device (10), comprising:
a catheter shaft (312) having an inner surface defining a lumen;
a liner (326) disposed within the lumen and along the inner surface;
wherein at least a portion of the liner (326) is radially spaced from the inner surface of the catheter shaft (312) so that a space (324) is defined therebetween;
wherein the liner (326) includes an inner layer (316), an intermediate layer (328), and an outer polymeric reinforcing member (318); and
wherein the outer polymeric reinforcing member (318) has a melting temperature lower than the melting temperature of the inner layer (316) and/or the intermediate layer (328).

12. The medical device (10) of claim 11, wherein the catheter shaft (312) has a plurality of slots formed therein.

13. The medical device (10) of any one of claims 11-12, wherein the liner (326) is free from attachment with the inner surface of the catheter shaft (312).

14. The medical device (10) of any one of claims 11-13, wherein the outer polymeric reinforcing member (318) includes a polymeric braid.

## Patentansprüche

1. Medizinische Vorrichtung (10), aufweisend:
einen Katheterschaft (112) mit einer Innenfläche, die ein Lumen definiert;
eine Einlage (126), die in dem Lumen und entlang der Innenfläche angeordnet ist;
wobei mindestens ein Abschnitt der Einlage (126) von der Innenfläche des Katheterschafts (112) radial beabstandet ist, so dass dazwischen ein Raum definiert wird;
wobei die Einlage (126) eine Innenschicht (116), eine Außenschicht und ein polymeres Verstärkungselement (118) aufweist;
wobei mindestens ein Teil des polymeren Verstärkungselements (118) zwischen einer Außenfläche der Innenschicht (116) und einer Außenfläche der Außenschicht angeordnet ist;
wobei das polymere Verstärkungselement (118) eine Schmelztemperatur hat, die niedriger als die Schmelztemperatur der Innenschicht (116) und/oder der Außenschicht ist; und
wobei das polymere Verstärkungselement (118) mindestens teilweise in die Außenschicht der Einlage (126) eingebettet ist.

2. Medizinische Vorrichtung (10) nach Anspruch 1, wobei der Katheterschaft (112) eine Nickel-Titan-Legierung aufweist.

3. Medizinische Vorrichtung (10) nach einem der Ansprüche 1-2, wobei in dem Katheterschaft (112) mehrere Schlitze ausgebildet sind.

4. Medizinische Vorrichtung (10) nach einem der Ansprüche 1-3, wobei die Einlage (126) frei von Befestigung an der Innenfläche des Katheterschafts (112) ist.

5. Medizinische Vorrichtung (10) nach einem der Ansprüche 1-4, wobei die Innenschicht (116) der Einlage (126) Polytetrafluorethylen aufweist.

6. Medizinische Vorrichtung (10) nach einem der Ansprüche 1-5, wobei die Innenschicht (116) der Einlage (126) fluoriertes Ethylenpropylen aufweist.

7. Medizinische Vorrichtung (10) nach einem der Ansprüche 1-6, wobei die Außenschicht der Einlage (126) Polyetherblockamid aufweist.

8. Medizinische Vorrichtung (10) nach einem der Ansprüche 1-7, wobei die Außenschicht der Einlage (126) eine Mischung aus Polyetherblockamid und Polyurethan aufweist.

9. Medizinische Vorrichtung (10) nach einem der Ansprüche 1-8, wobei das polymere Verstärkungselement (118) ein Polymergeflecht aufweist.

10. Medizinische Vorrichtung (10) nach einem der Ansprüche 1-9, wobei das polymere Verstärkungselement (118) Polyethylengeflecht mit ultrahohem Molekulargewicht aufweist.

11. Medizinische Vorrichtung (10), aufweisend:
einen Katheterschaft (312) mit einer Innenfläche, die ein Lumen definiert;
eine Einlage (326), die in dem Lumen und entlang der Innenfläche angeordnet ist;
wobei mindestens ein Teil der Einlage (326) von der Innenfläche des Katheterschafts (312) radial beabstandet ist, so dass dazwischen ein Raum (324) definiert wird;
wobei die Einlage (326) eine Innenschicht (316), eine Zwischenschicht (328) und ein äußeres polymeres Verstärkungselement (318) umfasst; und
wobei das äußere polymere Verstärkungselement (318) eine Schmelztemperatur hat, die niedriger als die Schmelztemperatur der Innenschicht (316) und/oder der Zwischenschicht (328) ist.

12. Medizinische Vorrichtung (10) nach Anspruch 11, wobei in dem Katheterschaft (312) mehrere Schlitze ausgebildet sind.

13. Medizinische Vorrichtung (10) nach einem der Ansprüche 11-12, wobei die Einlage (326) frei von Befestigung an der Innenfläche des Katheterschafts (312) ist.

14. Medizinische Vorrichtung (10) nach einem der Ansprüche 11-13, wobei das polymere äußere Verstärkungselement (318) ein Polymergeflecht aufweist.

## Revendications

1. Dispositif médical (10), comprenant :
une tige de cathéter (112) ayant une surface interne définissant une lumière ;
une chemise (126) disposée à l'intérieur de la lumière et le long de la surface interne ;
où au moins une section de la chemise (126) est radialement distante de la surface interne de la tige de cathéter (112) de sorte qu'un espace est défini entre elles ;
où la chemise (126) inclut une couche interne (116), une couche externe et un élément de renforcement polymérique (118) ;
où au moins une partie de l'élément de renforcement polymérique (118) est disposée entre une surface externe de la couche interne (116) et une surface externe de la couche externe ;
où l'élément de renforcement polymérique (118) a une température de fusion plus basse que la température de fusion de la couche interne (116) et/ou la couche externe ; et
où l'élément de renforcement polymérique (118) est inclus au moins en partie dans la couche externe de la chemise (126).

2. Dispositif médical (10) selon la revendication 1, où la tige de cathéter (112) inclut un alliage nickel-titane.

3. Dispositif médical (10) selon l'une quelconque des revendications 1-2, où la tige de cathéter (112) a une pluralité de fentes formées dans celle-ci.

4. Dispositif médical (10) selon l'une quelconque des revendications 1-3, où la chemise (126) est dépourvue de fixation avec la surface interne de la tige de cathéter (112).

5. Dispositif médical (10) selon l'une quelconque des revendications 1-4, où la couche interne (116) de la chemise (126) inclut du polytétrafluoroéthylène.

6. Dispositif médical (10) selon l'une quelconque des revendications 1-5, où la couche interne (116) de la chemise (126) inclut de l'éthylène propylène fluoré.

7. Dispositif médical (10) selon l'une quelconque des revendications 1-6, où la couche externe de la chemise (126) inclut du polyétheramide séquencé.

8. Dispositif médical (10) selon l'une quelconque des revendications 1-7, où la couche externe de la chemise (126) inclut un mélange de polyétheramide séquencé et de polyuréthane.

9. Dispositif médical (10) selon l'une quelconque des revendications 1-8, où l'élément de renforcement polymérique (118) inclut une tresse polymérique.

10. Dispositif médical (10) selon l'une quelconque des revendications 1-9, où l'élément de renforcement polymérique (118) inclut une tresse de polyéthylène de masse moléculaire ultra-haute.

11. Dispositif médical (10), comprenant :
une tige de cathéter (312) ayant une surface interne définissant une lumière ;
une chemise (326) disposée à l'intérieur de la lumière et le long de la surface interne ;
où au moins une partie de la chemise (326) est radialement distante de la surface interne de la tige de cathéter (312) de sorte qu'un espace (324) est défini entre elles ;
où la chemise (326) inclut une couche interne (316), une couche intermédiaire (328) et un élément de renforcement polymérique externe (318) ; et
où l'élément de renforcement polymérique externe (318) a une température de fusion plus basse que la température de fusion de la couche interne (316) et/ou la couche intermédiaire (328).

12. Dispositif médical (10) selon la revendication 11, où la tige de cathéter (312) a une pluralité de fentes formées dans celle-ci.

13. Dispositif médical (10) selon l'une quelconque des revendications 11-12, où la chemise (326) est dépourvue de fixation avec la surface interne de la tige de cathéter (312).

14. Dispositif médical (10) selon l'une quelconque des revendications 11-13, où l'élément de renforcement polymérique externe (318) inclut une tresse polymérique.
